# EUROPÄISCHE PATENTSCHRIFT

(11) **EP 2 572 738 B1**
(45) Veröffentlichungstag und Bekanntmachung des Hinweises auf die Patenterteilung: **08.11.2017**
(21) Anmeldenummer: 12182753.9
(22) Anmeldetag: 03.09.2012
(51) Int. Cl.: A61L 27/46, A61C 8/02, A61F 2/28

(54) **Formkörper mit kollagenhaltigem Kompositmaterial zum Einbringen in eine Knochendefektstelle**
Mould with composite material containing collagen for insertion in a bone defect point
Corps de formage avec matériau composite contenant du collagène destiné à être introduit à l'emplacement d'un défaut osseux

(30) Priorität: 19.09.2011 DE 102011082960
(43) Veröffentlichungstag der Anmeldung: 27.03.2013
(73) Patentinhaber: Resorba Medical GmbH, 90745 Nürnberg (DE)
(72) Erfinder: Frey, Markus, 90429 Nürnberg (DE); Ahlers, Dr. Michael, 90547 Stein (DE); Sorg, Dr. Karl-Heinz, 93333 Neustadt/Donau (DE); Huber, Christian, 90480 Nürnberg (DE)
(74) Vertreter: Rau, Schneck & Hübner Patentanwälte Rechtsanwälte PartGmbB

(56) Entgegenhaltungen:
- DE-A1-102006 047 054
- DE-B3-102008 010 893

## Beschreibung

Die Erfindung betrifft einen Formkörper zum Einbringen in einen Alveolarraum oder in eine andere Knochendefektstelle zur Wundheilung sowie zur Knochenregeneration und -konservierung.

Bei der medizinischen Behandlung kann es zu (kleineren) iatrogenen Knochendefekten kommen. Ein Beispiel hierfür ist die Wundhöhle nach dem Ausräumen einer Knochenzyste. Auch die nach einer Zahnextraktion vorhandene Zahntasche (= Alveole) lässt sich als Spezialfall eines solchen Knochendefekts verstehen.

Nach einer Zahnextraktion sind in der Zahnmedizin verschiedene Behandlungsverfahren bekannt. Bei einem ersten Ansatz erfolgt zunächst eine Ausheilung der Alveole, um danach mit einer Zahnersatz-Behandlung für z.B. eine Zahnimplantation neu zu beginnen. Hierbei kann es während der Abheilzeit zu einem unerwünschten Schwund an Knochensubstanz kommen. Ursache hierfür ist z.B. eine Entzündung in der Alveole, die die fibrinolytische Aktivität des Blutes erhöht, weshalb sich kein stabiles Blutgerinnsel (= Koagulum) ausbilden kann. Es kommt zu einer gestörten Wundheilung und einem Verlust von Knochengewebe. Als Folge davon lässt sich der Zahnersatz entweder nicht mehr optimal verankern oder es muss sogar eine aufwändige Regenerationsbehandlung des Kieferknochens zwischengeschaltet werden.

Bei einem anderen Ansatz wird unmittelbar nach der Zahnextraktion ein Implantat eingesetzt. Ein Beispiel für ein derartiges Implantat ist z.B. in der DE 196 30 034 A1 beschrieben. Es enthält einen harten Titan-Kern, der unter anderem mit einer kollagenhaltigen Beschichtung versehen ist und der nach einer Einwachsphase als Basis für eine Zahnkrone dient. Bei diesen unmittelbar eingesetzten Implantaten kann es z.B. wegen Entzündungen in der Alveole zu Problemen beim Einwachsen kommen.

Weiterhin ist aus der DE 10 2006 047 054 A1 ein Implantatlager bekannt, das patientenindividuell entsprechend der Größe der Alveole angefertigt wird. Aufgrund der individuellen Sonderanfertigung resultiert ein vergleichsweise hoher Fertigungs- und Kostenaufwand. Das Implantatlager besteht aus Keramik, vorzugsweise aus dem synthetischen Knochenersatzmaterial Hydroxylapatit, und hat eine nach innen abnehmende Dichte bzw. zunehmende Porosität. Es hat eine sehr feste Stützstruktur und dient nach dem Verwachsen mit dem Kieferknochen als unmittelbare Basis, in der das Zahnimplantat verankert wird. Ein Einwachsen des natürlichen Knochens in das Zahnimplantat wird bei Verwendung des Implantatlagers erschwert und verlangsamt. Dieses Implantatlager stellt einen dauerhaften avitalen Knochenersatz dar. Zur besseren Fixierung wird es mittels Schrauben am Kiefer befestigt. Dieses Implantatlager liegt sehr eng an der Alveolenwand an und kann dort beim Einsetzen zu zusätzlichen Verletzungen führen, wodurch Entzündungen und eine verzögerte Wundheilung auftreten können.

Außerdem ist aus der DE 10 2008 010 893 B3 und aus der EP 2 249 739 B1 ein zum Einsetzen in die Alveole bestimmter resorbierbarer Verbundkörper aus einem Grundkörper und einer Abdeckmembran bekannt. Der Grundkörper kann aus einem Kollagenmaterial bestehen, in dem ein Kern aus Knochenersatzmaterial vollständig eingebettet ist oder in das Knochenersatzmaterial homogen verteilt eingelagert ist. Der Verbundkörper dient z.B. nach einer Zahnextraktion zur Knochenregeneration und zum Erhalt der vorhandenen Knochensubstanz, um später nach erfolgter Wundheilung ein Zahnimplantat in dem Kieferknochen verankern zu können. Das Knochenersatzmaterial ist relativ hart und unflexibel, so dass es beim Einsetzen des Verbundkörpers in die Alveole ebenfalls zu zusätzlichen Verletzungen mit den oben genannten Folgen kommen kann.

In der DE 10 2007 012 276 A1 wird außerdem eine Zusammensetzung zur Behandlung von Knochen- und Knorpeldefekten beschrieben. Die Zusammensetzung enthält mindestens ein Kollagen, welches mindestens einen osteoinduktiven oder chondroinduktiven Wirkstoff aufweist, sowie mindestens einen Zusatz aus einem Differenzierungs- und/oder Wachstumsfaktor. Weiterhin kann die Zusammensetzung optional ein Füllmaterial enthalten. Das Füllmaterial kann als Knochenersatzmaterial verstanden werden. Als Füllmaterialien werden u.a. Kollagen des Typs 1, Extrake aus nativem Knochen, und keramische Werkstoffe, wie z.B. Tricalciumphosphat und Hydroxylapatit, genannt. Die Zusammensetzung kann als im Wesentlichen formstabiler Körper vorliegen. Über die Verteilung des Füllmaterials innerhalb der Zusammensetzung werden keine Angaben gemacht.

Die Aufgabe der Erfindung besteht darin, einen Formkörper der eingangs bezeichneten Art anzugeben, der eine verbesserte Wundheilung sowie eine verbesserte Knochenregeneration und -konservierung bewirkt.

Zur Lösung dieser Aufgabe wird ein Formkörper entsprechend den Merkmalen des Patentanspruchs 1 angegeben. Für den Formkörper ist ein Kompositmaterial mit mindestens einer ersten Materialkomponente in Form eines Kollagenmaterials und einer zweiten Materialkomponente in Form eines Knochenersatzmaterials vorgesehen. Das Kollagenmaterial bildet eine Matrix, in die das Knochenersatzmaterial inhomogen verteilt eingebettet ist, so dass ein auf ein beliebiges Teilvolumen von 30 mm³ bezogener lokaler Kollagenanteil überall mindestens 10 % beträgt und ein auf das Gesamtvolumen bezogener globaler Kollagenanteil im Bereich zwischen 30 % und 95 % liegt. Der Formkörper ist komprimierbar und hat bei einer Kompression um bis zu 40 % seines Ausgangsvolumens ein Kompressionsmodul von höchstens 1,0 MPa. Ein jeweils auf ein Teilvolumen von 30 mm³ bezogener lokaler Knochenersatzmaterialanteil hat an einer bei der Applikation der Öffnung der Knochendefektstelle zugewandten oberen Randfläche seinen maximalen Wert und nimmt ausgehend von der oberen Randfläche nach innen ab, wobei sich eine mit Knochenersatzmaterial versehene KEM-Zone ausgehend von der oberen Randfläche ins Innere erstreckt. Es ist eine knochenersatzmaterialfreie Zone vorhanden, die an eine der oberen Randfläche gegenüberliegende untere Randfläche angrenzt.

Der erfindungsgemäße Formkörper ist zum Einsetzen in eine Knochendefektstelle, die sich insbesondere im Gesichtsbereich, vorzugsweise im Mund- oder Kiefer-Bereich, befinden kann, bestimmt. Bei Ausgestaltungen, bei denen ein Einbringen in einen Alveolarraum vorgesehen ist, kann der Formkörper insbesondere auch als Alveolarfüllkörper oder Dentalfüllkörper bezeichnet werden.

Der insbesondere aus dem kollagenhaltigen Kompositmaterial bestehende Formkörper unterstützt durch seinen kombinierten Aufbau aus Kollagen und Knochenersatzmaterial die natürliche Knochenregeneration. Das Kollagen dient als regenerative Leitschiene, das Knochenmaterial als stabilisierende Komponente, wobei beide Komponenten synergistisch zusammenwirken können. Bei einem Einsatz als Alveolarfüllkörper ist der erfindungsgemäße Formkörper im Gegensatz zu dem Implantatlager gemäß der DE 10 2006 047 054 A1 keine primäre Basis für die Verankerung eines Implantats, sondern dient vielmehr im Vorfeld einer Zahnimplantation durch zumindest teilweise Regeneration der natürlichen, physiologischen Knochenmatrix erst zum Aufbau einer geeigneten Implantatbasis. Ein später eingesetztes Implantat wächst dann in eine vitale, physiologische Knochenbasis ein. Eine avitale, synthetische Basis wie das Implantatlager gemäß der DE 10 2006 047 054 A1 zeigt dagegen keine vergleichbaren günstigen Eigenschaften für das Einwachsen des Implantats.

Soll bei einem anderen Anwendungsfall ein Implantat direkt in den vorhandenen Kieferknochen eingesetzt werden, können um das Implantat herum verbliebene Hohlräume noch in derselben Behandlungssitzung, in der auch das Implantat eingesetzt wird, mit mindestens einem erfindungsgemäßen Formkörper gefüllt werden. Dadurch verkürzt sich die Behandlungsdauer. Der oder die in die Hohlräume eingesetzte/n Formkörper fördern die Wundheilung und das Einwachsen des Implantats.

Das Knochenersatzmaterial führt nach dem Einbringen zu einer relativ raschen Stabilisierung des Knochendefekts. Eine vollständige Knochenregenation wird allerdings erst nach der vergleichsweise langsamen Resorption des Knochenersatzmaterials erreicht. Zu viel eingebrachtes Knochenersatzmaterial belastet somit den Organismus, behindert die Vaskularisation und verzögert den Heilungsprozess. Aufgrund der inhomogenen Verteilung des Knochenersatzmaterials (= KEM) innerhalb der Kollagenmatrix kann die Belastung mit Knochenersatzmaterial reduziert werden, ohne auf dessen positive mechanisch stabilisierende Wirkungen zu verzichten, insbesondere an den Stellen, an denen eine solche Stabilisierung benötigt wird. Es gibt aber andererseits auch Bereiche innerhalb des Knochendefekts, in denen keine Unterstützung notwendig ist, beispielsweise am Alveolenboden. Mittels der inhomogenen Verteilung des Knochenersatzmaterials innerhalb der Kollagenmatrix lässt sich der Einsatz des Knochenersatzmaterials bedarfsgerecht und an den Anwendungszweck angepasst dosieren. Die Menge des applizierten Knochenersatzmaterials kann somit auf das notwendige Maß reduziert werden. Innerhalb des Formkörpers gibt es Zonen mit wenig oder gar keinem Knochenersatzmaterial. Der Formkörper kann also insbesondere auch mindestens eine knochenersatzmaterialfreie Zone enthalten. Letztere kann durchaus sehr klein sein und z.B. nur etwa 10 % des Gesamtvolumens des Formkörpers ausmachen. Insbesondere liegt der auf das Gesamtvolumen bezogene prozentuale Anteil der knochenersatzmaterialfreien Zone im Bereich von 30 % bis 90%, vorzugsweise von 40 % bis 85 % und bevorzugt von 50 % bis 75 %.

Grundsätzlich ist aber auch eine Ausgestaltung möglich, bei der überall im Formkörper ein gewisser, wenn auch örtlich voneinander abweichender Anteil an Knochenersatzmaterial vorgesehen ist. So kann z.B. ein fließender oder kontinuierlicher Verlauf des Anteils an eingelagertem Knochenersatzmaterial vorgesehen sein, ohne dass ein lokaler Knochenersatzmaterialanteil irgendwo innerhalb des Formkörpers komplett zu Null wird.

Der globale Kollagenanteil insbesondere jeweils zwischen 55 % und 95 %, vorzugsweise zwischen 60 % und 90 %, und bevorzugt zwischen 70 % und 80%.

Das Kollagen hat ebenfalls eine günstige Wirkung auf die Knochenregenration. Kollagen ist im Gegensatz zu dem Knochenersatzmaterial ein weiches, plastisch verformbares Material. Es trägt weniger zur mechanischen Stabilisierung bei. Dafür beeinträchtigt es aufgrund seiner sehr leichten Resorbierbarkeit die natürliche Knochenregeneration (Osteogenose und Vaskularisation) deutlich weniger als das Knochenersatzmaterial.

Mit dem kombinierten Aufbau des Formkörpers aus Kollagen und Knochenersatzmaterial kann man sich die Vorteile beider Materialien zunutze machen. Bei einer bevorzugten Ausführungsform ist für den Formkörper ein Kompositmaterial vorgesehen, das ausschließlich aus dem Kollagenmaterial und dem Knochenersatzmaterial besteht. Andere Ausführungsformen mit zusätzlichen Materialien und/oder Stoffen innerhalb des Kompositmaterials sind aber grundsätzlich ebenfalls möglich.

Vorteilhafterweise ist der Formkörper insgesamt komprimierbar. Insbesondere lässt sich der Formkörper deutlich besser komprimieren als vergleichbare bisher bekannte Ausgestaltungen, wie z.B. das Implantatlager gemäß der DE 10 2006 047 054 A1, bei dem praktisch gar keine Kompression möglich ist. Die inhomogene Einbettung des Knochenersatzmaterials in die Kollagenmatrix verbessert die Komprimierbarkeit des Formkörpers, insbesondere in den Bereichen, in denen nur wenig oder gar kein Knochenersatzmaterial vorgesehen ist. Die Komprimierbarkeit des Formkörpers erleichtert das Einsetzen des Formkörpers in die Wundhöhle (einfaches Zusammendrücken zwischen Daumen und Zeigefinger, dann Einsetzen in die Wundhöhle) und ermöglicht ein besseres und schonenderes Anschmiegen an die Wand der Wundhöhle, z.B. der Alveole, ohne dass dabei zusätzliche Verletzungen auftreten. Dadurch resultiert eine verbesserte Wundheilung und Knochenregeneration bzw. -konservierung.

Der Formkörper ist durch den (Zahn-)Arzt während der Behandlungssitzung mit geringem Aufwand an die jeweilige Knochendefektstelle anpassbar. Dies wird zum einen durch die Komprimierbarkeit erreicht. Zum anderen ist der Formkörper insbesondere auch schneidbar. Der Zuschnitt des Formkörpers kann vorzugsweise mittels eines üblichen Schneidwerkzeugs, wie z.B. eines Messers oder eines Skalpells, erfolgen. Ein Spezialwerkzeug ist hierfür entbehrlich. Auch die einfache und gute Anpassbarkeit des Formkörpers an die Knochendefektstelle führt letztendlich zu einer sehr guten Wundheilung, Knochenregeneration und -konservierung.

Aufgrund der an der Randfläche maximalen und nach innen hin abnehmenden Knochenersatzmaterial-Konzentration wird das Knochenersatzmaterial gezielt vor allem dort, nämlich insbesondere im Öffnungsbereich einer Knochendefektstelle, eingebracht, wo seine mechanisch stützende Funktion besonders gefragt ist.

Insgesamt schafft der erfindungsgemäße Formkörper einen "Regenerativen Raum", aufgrund dessen die physiologische Wundheilung gefördert und ein Verlust von Knochenmasse vermieden wird.

Vorteilhafte Ausgestaltungen des erfindungsgemäßen Formkörpers ergeben sich aus den Merkmalen der von Anspruch 1 abhängigen Ansprüche.

Günstig ist eine Ausgestaltung, bei der der Formkörper bei einer Kompression um bis zu 40 % seines Ausgangsvolumens ein Kompressionsmodul von höchstens 0,5 MPa, vorzugsweise von höchstens 0,25 MPa, hat. Die Kompression erfolgt also auf bis zu 60 % des Ausgangsvolumens des Formkörpers. Damit weist der Formkörper ein annähernd gleich günstiges Kompressionsverhalten auf wie ein vollständig aus Kollagenmaterial bestehender Vergleichformkörper. Für eine Kompression des Formkörpers um bis zu 30 % seines Ausgangsvolumens, also auf bis zu 70 % seines Ausgangsvolumens, ist insbesondere höchstens eine doppelt so große, vorzugsweise sogar nur im Wesentlichen die gleiche, Druckkraft erforderlich wie bei dem vollständig aus Kollagenmaterial bestehenden Vergleichsformkörper. Für eine Kompression des Formkörpers um bis zu 40 % seines Ausgangsvolumens ist insbesondere eine Druckkraft von höchstens 50 N, insbesondere von höchstens 25 N, vorzugsweise von höchstens 10 N erforderlich. Außerdem ist die Kompressionsverformung des Formkörpers vorteilhafterweise plastisch. Die Kompressionsverformung bildet sich also nach dem Einsetzen in die Knochendefektstelle, zumindest soweit es die örtlichen Gegebenheiten zulassen, wieder zurück und schmiegt sich dabei an die Wände der Knochendefektstelle an. Dadurch werden Hohlräume weitestgehend vermieden, was sich günstig auf die Wundheilung und die Knochenregenration auswirkt.

Gemäß einer weiteren günstigen Ausgestaltung liegt der maximale Wert des auf ein Teilvolumen von 30 mm³ bezogenen lokalen Knochenersatzmaterialanteils an der oberen Randfläche zwischen 10 % und 90 %. Insbesondere kann der maximale lokale Knochenersatzmaterialanteil an einem auf die gesamte obere Randfläche bezogenen Flächenanteil von zwischen 10 % und 100 % vorliegen. Auch dadurch lässt sich die Menge des im jeweiligen Anwendungsfall eingebrachten Knochenersatzmaterials individuell an die fallspezifischen Anforderungen anpassen.

Günstig ist eine weitere Ausgestaltung, bei der die Abnahme des Knochenersatzmaterialanteils in der KEM-Zone kontinuierlich oder mit mindestens einer diskreten Abstufung, vorzugsweise mit mehreren diskreten Abstufungen, oder bereichsweise kontinuierlich und bereichsweise abgestuft erfolgt, insbesondere bis zu einem minimalen lokalen Knochenersatzmaterialanteil zwischen 1 % und 40 %. Auch mittels dieser Designmaßnahmen kann eine sehr genaue Anpassung an die jeweiligen Bedürfnisse erfolgen.

Gemäß einer weiteren günstigen Ausgestaltung hat der Formkörper eine längliche Grundform mit einer Längsachse und mit einer senkrecht zur Längsachse angeordneten stirnseitigen Begrenzungsfläche. Die stirnseitige Begrenzungsfläche, bei der es sich bei der Applikation um die obere, d.h. der Öffnung der Knochendefektstelle zugewandte, Stirnfläche handelt, ist dann diejenige Randfläche, von der ausgehend der lokale Knochenersatzmaterialanteil abnimmt. Ein solcher länglicher Formkörper eignet sich sehr gut als Alveolarfüllkörper, d.h. für einen Einsatz zur Behandlung der Wundhöhle nach einer Zahnextraktion. Bei diesem Anwendungsfall ist es vorteilhaft, wenn das Knochenersatzmaterial hauptsächlich oben an der Öffnung der Alveole eingebracht wird und die Knochenersatzmaterial-Konzentration zum Alveolenboden hin abnimmt.

Günstig ist eine weitere Ausgestaltung, bei der die KEM-Zone längs ihrer Ausdehnungsrichtung ins Innere bezogen auf eine mit Knochenersatzmaterial versehene KEM-Ausgangsfläche der oberen Randfläche senkrecht zur Ausdehnungsrichtung Querschnittsflächen hat, die größer oder kleiner als die KEM-Ausgangsfläche oder gleich groß wie die KEM-Ausgangsfläche sind. Die KEM-Zone kann sich also innerhalb des Formkörpers insbesondere aufweiten, verengen oder gleich groß bleiben. Die diesbezüglich am besten geeignete Ausgestaltung lässt sich ohne weiteres entsprechend den spezifischen Anforderungen wählen. Besonders günstig sind Ausgestaltungen, deren KEM-Zone sich längs ihrer Ausdehnungsrichtung ins Innere verengt. Insbesondere eine Ausgestaltung mit einer KEM-Zone in trichterartiger Form, vorzugsweise in Form eines Teils eines Rotationshyperboloids, ergibt für die alveolare Anwendung eine besonders vorteilhafte Kombination aus Komprimierbarkeit im unteren Teil und eine nach oben zunehmende Unterstützung der Alveole durch Knochenersatzmaterial. Dies gilt auch für eine alternative Ausgestaltung mit tropfenförmiger KEM-Zone, die sich ebenfalls zumindest in gewissem Umfang in Ausdehnungsrichtung verengt.

Gemäß einer weiteren günstigen Ausgestaltung hat die KEM-Zone im Wesentlichen eine tropfenartige Form oder im Wesentlichen ein trichterartige Form, insbesondere eine Kegel- oder Kegelstumpfform, oder zumindest teilweise die Form eines Teils eines Rotationshyperboloids. Mittels dieser verschiedenen Ausgestaltungen kann man praktisch alle denkbaren Anwendungsfälle sehr gut abdecken. Bei der rotationshyperboloiden Ausgestaltung hat die KEM-Zone insbesondere im Wesentlichen die Form der oberen Hälfte eines Rotationshyperboloids. Außerdem kann die Gestalt der KEM-Zone in dieser Ausgestaltung von der mathematisch exakten Form eines (Teils des) Rotationshyperboloids etwas abweichen, insbesondere an den oberen und unteren Randflächen der KEM-Zone. Wie bereits erwähnt, eignen sich die Ausgestaltungen mit einer KEM-Zone in Form eines Teils eines Rotationshyperboloids und mit einer tropfenförmigen KEM-Zone besonders gut für einen alveolaren Einsatz

Gemäß einer weiteren günstigen Ausgestaltung unterscheiden sich das Kollagenmaterial und das Knochenersatzmaterial erkennbar voneinander, wobei insbesondere mindestens ein Material aus der Gruppe von dem Kollagenmaterial und dem Knochenersatzmaterial gefärbt ist. Dann kann der behandelnde Arzt die Lage des inhomogen im Formkörper eingebetteten Knochenersatzmaterials besser erkennen. Dies erleichtert die Handhabung, insbesondere den korrekten Zuschnitt des Formkörpers (falls ein solches Zuschneiden erforderlich sein sollte) und das richtig orientierte Einsetzen des Formkörpers in die Knochendefektstelle.

Günstig ist eine weitere Ausgestaltung, bei der das Knochenersatzmaterial aufbereitetes natives Knochenmaterial, insbesondere gereinigtes Spongiosamaterial, oder synthetisches Knochenersatzmaterial, insbesondere Tricalciumphosphat-Granulat, Hydroxylapatit-Granulat, resorbierbares Biokeramik-Granulat, wie z.B. Bioglas-Granulat, biphasisches Knochenersatzmaterial-Granulat oder multiphasisches Knochenersatzmaterial-Granulat, ist. Biphasisches Knochenersatzmaterial-Granulat ist ein Komposit aus zwei synthetischen Knochenersatzmaterialien, multiphasisches Knochenersatzmaterial ein Komposit aus mehr als zwei synthetischen Knochenersatzmaterialien. Die Zubereitungen der Knochenersatzmaterial-Granulate können außerdem auch weitere bioresorbierbare Polymere enthalten, wie z.B. Bindemittel in Form von Polylactid. Das Knochenersatzmaterial ist jeweils insbesondere körnig und hat einen bevorzugten Partikeldurchmesser zwischen 0,05 mm und 3 mm, insbesondere zwischen 0,1 mm und 2 mm, vorzugsweise zwischen 0,2 mm und 1 mm. Knochenersatzmaterial gemäß diesen Vorgaben hat jeweils eine sehr gute mechanische stabilisierende Wirkung und fördert die Knochenbildung in der Knochendefektstelle während des Wundheilungsprozesses.

Günstig ist eine Variante, bei der das Kollagenmaterial zumindest teilweise aus einem porösen Kollagen, z.B. aus lyophilisiertem, getrocknetem oder mittels Verfilzen hergestelltem Kollagen, vorzugsweise aus rekonstituiertem Kollagen vom Typ 1 besteht. Vorteilhafterweise ist das Kollagenmaterial equinen Ursprungs. Weiterhin ist es bevorzugt vorgesehen, dass das Kollagenmaterial eine Dichte von 1 bis 25 mg/cm³, vorzugsweise von 5 bis 12 mg/cm³, hat. Kollagen mit diesen Dichtewerten lässt sich besonders gut herstellen. Kollagen ist als bioresorbierbares Material sehr gut verträglich und findet Anwendung zur Blutstillung, zur Füllung von Knochen und Gewebedefekten und zur Abdeckung von Wunden. Kollagen unterstützt die Blutstillung, indem Thrombozyten an den Kollagenfibrillen aggregieren und sich ein Koagulum bildet. Durch die Wirkung von eingewanderten Makrophagen und körpereigener Kollagenase wird das Kollagen im Rahmen der Wundheilung komplett resorbiert. Günstig ist auch eine Variante bei der zumindest teilweise besonders hydrophiles, leicht benetzbares Kollagenmaterial eingesetzt wird. Dieses hydrophilere Kollagenmaterial lässt sich durch eine besondere Rohstoffauswahl oder den Zusatz von hydrophilen Substanzen herstellen.

Günstig ist eine weitere Ausgestaltung, bei der der Formkörper eine im Wesentlichen längliche Grundform mit einer Längsachse hat, wobei die Ausdehnung in Richtung der Längsachse zwischen 0,4 cm und 3 cm und die maximale Ausdehnung senkrecht zur Längsachse zwischen 0,3 cm und 2,9 cm liegt. Längliche Formkörper mit diesen Abmessungen eignen sich sehr gut zum Einbringen in einen Alveolarraum. Auf einen zusätzlichen Zuschnitt kann dann in vielen Fällen verzichtet werden.

Gemäß einer anderen günstigen Ausgestaltung hat der Formkörper eine im Wesentlichen würfelförmige Grundform, wobei die Ausdehnung in Richtung der drei Würfelachsen jeweils zwischen 0,3 cm und 3 cm liegt. Solche Formkörper eignen sich besonders gut zum Auffüllen von u.U. von außen schwer zugänglichen Hohlräumen innerhalb von Knochendefektstellen, in die bereits ein anderes (Funktions-)Element, wie z.B. ein Implantat, eingebracht worden ist.

Günstig ist eine weitere Ausgestaltung, bei der der Formkörper ein Verbundkörper mit einem ersten Teilkörper aus dem Kompositmaterial mit inhomogen verteilt in eine Kollagenmatrix eingelagertem Knochenersatzmaterial und mit einem fest mit dem ersten Teilkörper verbundenen, als Abdeckmembran ausgebildeten zweiten Teilkörper ist. Eine Membranfläche des zweiten Teilkörpers ist größer als eine Grundfläche des ersten Teilkörpers. Der zweite Teilkörper ist so auf den ersten Teilkörper aufgesetzt, dass der zweite Teilkörper seitlich überall über die Grundfläche des ersten Teilkörpers hinausragt. Die Anordnung einer seitlich überstehenden Abdeckmembran auf einem Grundkörper (= erster Teilkörper) ist bereits aus der DE 10 2008 010 893 B3 bekannt. Die dort hinsichtlich der Abdeckmembran beschriebenen Vorteile und Ausgestaltungen ergeben sich gleichermaßen bei einer Kombination eines Grundkörpers, der aus dem Kompositmaterial mit inhomogen verteilt in eine Kollagenmatrix eingelagertem Knochenersatzmaterial hergestellt ist, mit der Abdeckmembran. Insbesondere deckt die Abdeckmembran die Alveole mit ihrem seitlich über den Grundkörper überstehenden Randbereich ab und bildet so eine wirksame Barriere gegen unkontrollierte Binde- und Epithelgewebeproliferation in die Alveole. Da Knochengewebe im Vergleich zum Binde- und Epithelgewebe wesentlich langsamer proliferiert, bestünde ohne die Abdeckmembran die Gefahr, dass das Binde- und Epithelgewebe den Alveolarraum schneller ausfüllt und damit das Knochenwachstum beeinträchtigt.

Gemäß einer weiteren bevorzugten Ausgestaltung enthält das Kompositmaterial einen wundheilenden, die Angiogenese fördernden oder das Knochenwachstum fördernden bioaktiven Bestandteil. Bei diesem bioaktiven Bestandteil handelt es sich vorzugsweise um ein natives isoliertes oder biotechnologisch gewonnenes Protein, nämlich BMP-2 (= Bone Morphogenic Protein 2). Hierunter fällt unter anderem auch TGF beta (TGF = Transforming Growth Factor) oder ähnliches. Auch fallen hierunter die Angiogenese fördernde Faktoren, wie z.B. nativ isoliertes oder biotechnologisch hergestelltes FGF-1 (Fibroblast Growth Factor 1), VEGF-A (Vascular Endothelial Growth Factor A) oder ähnliches.

Gemäß einer anderen ebenfalls günstigen Ausgestaltung enthält das Kompositmaterial einen antimikrobiellen oder antibiotisch wirkenden Bestandteil. Dieser antimikrobielle Bestandteil trägt dazu bei, Infektionen zu verhindern und/oder zu bekämpfen. Es handelt sich hierbei vorzugsweise um ein lokal verträgliches Antiseptikum, wie z.B. Polihexanid, Octenidin, Silberionen, Jodderivate, Chlorhexidin, Triclosan oder ähnliches. Ebenso kann eine für die lokale Anwendung geeignete antibiotisch wirkende Substanz, wie z.B. Gentamicin, Metronidazol, Vancomycin, Clindamycin oder ähnliches, zum Einsatz kommen.

Weitere Merkmale, Vorteile und Einzelheiten der Erfindung ergeben sich aus der nachfolgenden Beschreibung von Ausführungsbeispielen anhand der Zeichnungsfiguren. Es zeigen:
- Fig. 1 bis 11: Ausführungsbeispiele eines Formkörpers mit kollagenhaltigem Kompositmaterial zum Einbringen in eine Knochendefektstelle,
- Fig. 12: ein Diagramm mit Messkurven für Druckkraftverläufe in Abhängigkeit von der Kompression für verschiedene kollagenhaltige Formkörper, und
- Fig. 13: ein Ausführungsbeispiel für die Verwendung der Formkörper gemäß Fig. 1 bis 10 zum Auffüllen von Hohlräumen zwischen einem Implantat und den Alveolenwänden.

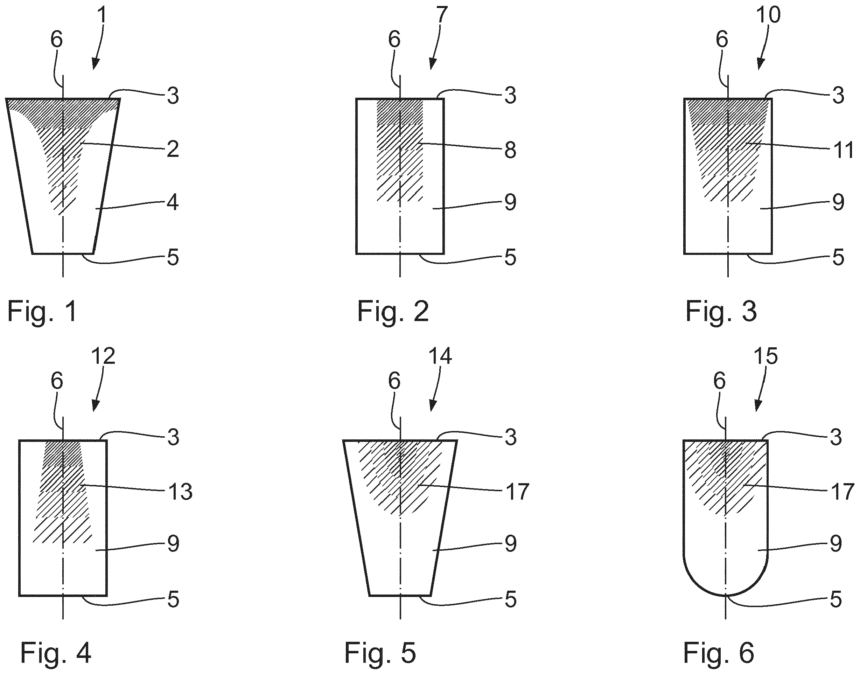

Einander entsprechende Teile sind in den Figuren 1 bis 13 mit denselben Bezugszeichen versehen. Auch Einzelheiten der im Folgenden näher erläuterten Ausführungsbeispiele können für sich genommen eine Erfindung darstellen oder Teile eines Erfindungsgegenstandes sein.

In Fig. 1 ist ein Formkörper 1 aus einem Kompositmaterial in einer Längsschnittdarstellung gezeigt. Der Formkörper 1 hat eine Kegelstumpfform. Das Kompositmaterial enthält eine Matrix aus Kollagenmaterial, in die bereichsweise, nämlich innerhalb einer KEM-Zone 2, Knochenersatzmaterial eingebettet ist. Die KEM-Zone 2 hat im Wesentlichen die Form der oberen Hälfte eines Rotationshyperboloids, wobei die KEM-Zone 2 am oberen und am unteren Ende von der mathematisch exakten hyperboloiden Form abweicht.

Das Knochenersatzmaterial ist innerhalb der KEM-Zone 2 inhomogen verteilt. Die maximale Knochenersatzmaterial-Konzentration befindet sich an einer oberen Randfläche 3, die bei dem gezeigten Ausführungsbeispiel des länglichen Formkörpers 1 die obere stirnseitige Begrenzungsfläche bildet. Die KEM-Zone 2 erstreckt sich ausgehend von der Randfläche 3 in das Innere des Formkörpers 1, wobei die Konzentration des Knochenersatzmaterials ausgehend von der Randfläche 3 nach Innen hin im Wesentlichen kontinuierlich abnimmt. In Fig. 1 ist lediglich zur besseren Darstellung ein gestufter Verlauf angedeutet. Tatsächlich verläuft die Konzentrationsabnahme des Knochenersatzmaterials aber eher fließend. Die KEM-Zone 2 füllt nicht den gesamten Formkörper 1 aus. Es gibt auch eine knochenersatzmaterialfreie Zone 4, die insbesondere auch an eine der oberen Randfläche 3 gegenüberliegende untere Randfläche 5 angrenzt.

Der Formkörper 1 weist einen im Wesentlichen bezogen auf eine Längsachse 6 rotationssymmetrischen Aufbau auf. Er hat eine Längsausdehnung in Richtung der Längsachse von 1,6 cm. An der oberen Randfläche 3 beträgt seine Ausdehnung senkrecht zur Längsachse 6, also der Durchmesser, etwa 1 cm.

Das Kollagenmaterial hat bei dem Ausführungsbeispiel eine Dichte von 11,2 mg/cm³. Der globale Kollagenanteil des gesamten Formkörpers 1 liegt bei etwa 80 %. Der lokale Kollagenanteil ist in der knochenersatzmaterialfreien Zone 4, die bei diesem Ausführungsbeispiel etwa 75 % des Gesamtvolumens des Formkörpers 1 einnimmt, naturgemäß besonders hoch, insbesondere bei annähernd 100%. Aber auch in der KEM-Zone 2 ist überall Kollagenmaterial vorhanden. Der auf ein beliebiges Teilvolumen von 30 mm³ bezogene lokale Kollagenanteil beträgt überall mindestens 10%. Dies gilt insbesondere auch in der an die obere Randfläche 3 angrenzenden Zone, innerhalb derer die Konzentration des Knochenersatzmaterials maximal ist.

Die KEM-Zone 2 enthält insgesamt 0,1 g inhomogen verteiltes Knochenersatzmaterial in Form von Tricalciumphosphat-Granulat. Die KEM-Zone 2 macht etwa 25 % des Gesamtvolumens des Formkörpers 1 aus. Der jeweils auf ein 30 mm³ großes Teilvolumen bezogene lokale Knochenersatzmaterialanteil hat mit etwa 40 % an der oberen Randfläche 3 seinen maximalen Wert.

Der Formkörper 1 ist insbesondere zum Einsetzen in eine Knochendefektstelle, insbesondere in eine Zahntasche (= Alveole) bestimmt, die sich beispielsweise nach einer Zahnextraktion bildet. Der Formkörper 1 ist also ein Alveolarfüllkörper, der bevorzugt so in die Alveole einzusetzen ist, dass die untere Randfläche 5 am Alveolenboden anliegt.

Das Einsetzen des Formkörpers 1 in die Alveole wird dadurch erleichtert, dass der Formkörper 1 komprimierbar ist. Die gute Komprimierbarkeit resultiert aus dem diesbezüglich besonders vorteilhaften Aufbau des Formkörpers 1 mit der weichen, plastisch verformbaren Kollagenmatrix, in die das mechanisch festere und weniger gut komprimierbare Knochenersatzmaterial inhomogen verteilt und mit einer lokalen Maximalkonzentration an einer Randfläche des Formkörpers 1 eingebettet ist.

Die beiden Materialkomponenten des Formkörpers 1 tragen jede auf ihre Weise zu einem besonders günstigen Heilungsverlauf und zu einer sehr guten Knochenregeneration bei. Es hat sich gezeigt, dass für eine derartig günstige Knochenregeneration gerade im Bereich des Alveolenbodens kein Knochenersatzmaterial benötigt wird. Dessen mechanisch stabilisierende Wirkung ist im Gegensatz zur Alveolenöffnung am Alveolenboden nicht erforderlich. Hier reicht zu Knochenregeneration das dort vorhandene Kollagenmaterial vollkommen aus. Das Kollagenmaterial wird verglichen mit dem Knochenersatzmaterial erheblich schneller resorbiert. Dadurch beschleunigen sich die Wundheilung und die Knochenregeneration. Andererseits ist die stabilisierende Wirkung des Knochenersatzmaterials an der Alveolenöffnung durchaus erwünscht und sinnvoll.

In Fig. 2 ist ein weiteres Ausführungsbeispiel eines Formkörpers 7 dargestellt, der ebenfalls aus dem kollagenhaltigen Kompositmaterial gefertigt ist. Auch der Formkörper 7 enthält eine KEM-Zone 8 mit einer inhomogen verteilten Einbettung von Knochenersatzmaterial innerhalb der Kollagenmatrix. Der Formkörper 7 hat ebenso wie die KEM-Zone 8 eine zylindrische Form. Die KEM-Zone 8 ist von einer knochenersatzmaterialfreien Zone 9 umgeben. Der maximale lokale Knochenersatzmaterialanteil der KEM-Zone 8 grenzt wiederum an die obere Randfläche 3 an, wobei sich die KEM-Zone 8 bei dem Formkörper 7 anders bei dem Formkörper 1 nicht über die gesamte Fläche der Randfläche 3, sondern nur über einen zentralen Bereich der Randfläche 3 erstreckt. Dieser zentrale Bereich beträgt etwa 60% der gesamten Randfläche 3. Der Anteil des Knochenersatzmaterials nimmt ausgehend von der Randfläche 3 innerhalb des Formkörpers 7 in Richtung der Längsachse 6 ab. Die Ausdehnung der KEM-Zone 8 senkrecht zur Längsachse 6 bleibt innerhalb des Formkörpers 7 im Wesentlichen gleich.

In Fig. 3 ist ein weiteres Ausführungsbeispiel eines Formkörpers 10 mit inhomogen in eine Kollagenmatrix eingebettetem Knochenersatzmaterial dargestellt. Der Formkörper 10 ist zylindrisch. Eine bei diesem Ausführungsbeispiel trichter- oder kegelstumpfförmige KEM-Zone 11 erstreckt sich ausgehend von der Randfläche 3 mit der maximalen Knochenersatzmaterial-Konzentration ins Innere des Formkörpers 10, wobei die maximale Ausdehnung der KEM-Zone 11 senkrecht zur Längsachse 6 an der oberen Randfläche 3 gegeben ist. Die Ausdehnung der KEM-Zone 11 senkrecht zur Längsachse 6 nimmt innerhalb des Formkörpers 10 ebenso ab wie der Anteil des Knochenersatzmaterials.

Ein Ausführungsbeispiel eines ähnlichen zylindrischen Formkörpers 12 ist in Fig. 4 dargestellt. Er enthält ebenfalls eine trichter- oder kegelstumpfförmige KEM-Zone 13, wobei der Öffnungswinkel der kegelstumpfförmigen KEM-Zone 13 genau in die entgegengesetzte Richtung orientiert ist wie bei der KEM-Zone 11 des Formkörpers 10. Dementsprechend hat die KEM-Zone 13 bei diesem Ausführungsbeispiel an der oberen Randfläche 3 die minimale Ausdehnung senkrecht zur Längsachse 6. Die Ausdehnung der KEM-Zone 13 senkrecht zur Längsachse 6 nimmt innerhalb des Formkörpers 12 zu. Der Anteil des Knochenersatzmaterials nimmt dagegen innerhalb des Formkörpers 12 ab.

In den Fig. 5 bis 7 sind weitere Ausführungsbeispiele für Formkörper 14 bis 16 aus kollagenhaltigem Kompositmaterial dargestellt. Diese Formkörper 14 bis 16 enthalten jeweils eine tropfenförmige KEM-Zone 17 mit einer maximalen Konzentration des Knochenersatzmaterials angrenzend an die obere Randfläche 3 im Bereich der Längsachse 6. Ausgehend von diesem Bereich mit maximaler Konzentration an Knochenersatzmaterial nimmt der Anteil an Knochenersatzmaterial sowohl parallel als auch senkrecht zur Richtung der Längsachse 6 ab. Der Formkörper 14 ist kegelstumpfförmig, wohingegen die Formkörper 15 und 16 eine zylindrische Grundform haben, die an dem der oberen Randfläche 3 gegenüberliegenden unteren Ende bei dem Formkörper 15 in eine Halbkugelform und bei dem Formkörper 16 in eine Kegelstumpfform übergeht.

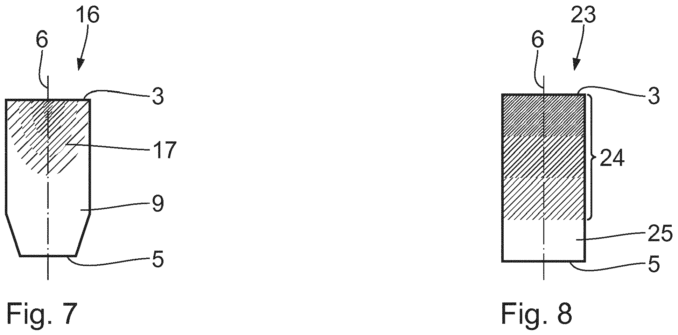

In Fig. 8 ist ein weiteres Ausführungsbeispiel eines kollagenhaltigen Formkörpers 23 dargestellt, der ebenfalls eine KEM-Zone 24 umfasst. Der Formkörper 23 ist zylindrisch, ebenso wie die KEM-Zone 24. Die KEM-Zone 24 grenzt bei diesem Ausführungsbeispiel vollflächig an die obere Randfläche 3 an und hat in diesem Bereich die maximale Knochenersatzmaterial-Konzentration. Im Unterschied zu den vorherigen Ausführungsbeispielen ist bei der KEM-Zone 24 keine kontinuierliche, sondern eine gestufte Abnahme der Knochenersatzmaterial-Konzentration vorgesehen. Anders als bei den vorherigen Ausführungsbeispielen entspricht der in Fig. 8 dargestellte gestufte Verlauf der Knochenersatzmaterial-Konzentration bei diesem Ausführungsbeispiel also den tatsächlichen Gegebenheiten. Die Abnahme erfolgt ausgehend von der Randfläche 3 nach Innen und in Richtung der Längsachse 6. Die KEM-Zone 24 umfasst beim gezeigten Ausführungsbeispiel drei Teilzonen mit jeweils innerhalb der Teilzone einheitlicher, aber untereinander unterschiedlicher Knochenersatzmaterial-Konzentration. Die KEM-Zone 24 geht an ihrem unteren Ende, d. h. an dem der Randfläche 3 gegenüberliegenden Ende, mit einem letzten Abstufungsschritt von der untersten Teilzone mit der niedrigsten Knochenersatzmaterial-Konzentration in eine knochenersatzmaterialfreie Zone 25 über.

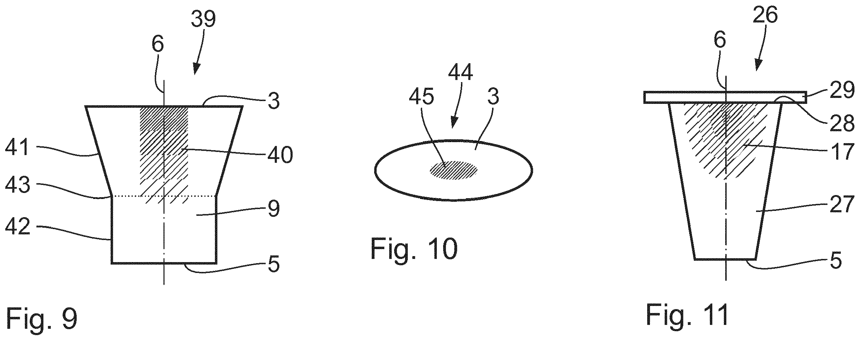

In Fig. 9 ist ein weiteres Ausführungsbeispiel eines Formkörpers 39 aus kollagenhaltigem Kompositmaterial dargestellt. Er umfasst eine zylindrische KEM-Zone 40 mit einer inhomogen verteilten Einbettung von Knochenersatzmaterial innerhalb der Kollagenmatrix. Die KEM-Zone 40 ist ähnlich aufgebaut wie die KEM-Zone 8 des Ausführungsbeispiels gemäß Fig. 2. Der Formkörper 39 hat aber eine etwas andere Außenkontur. Er setzt sich aus einem oberen kegelstumpfförmigen Teilabschnitt 41 und einem unteren zylindrischen Teilabschnitt 42 zusammen. Die beiden Teilabschnitte 41 und 42 grenzen an einer Stoßfläche 43 aneinander an. Die KEM-Zone 40 ist bei dem gezeigten Ausführungsbeispiel im Wesentlichen innerhalb des oberen Teilabschnitts angeordnet.

In Fig. 10 ist ein weiteres Ausführungsbeispiel eines kollagenhaltigen Formkörpers 44 gezeigt. Dargestellt ist hier kein Längsschnitt, sondern eine Draufsicht auf die obere Randfläche 3 des Formkörpers 44. Auch der Formkörper 44 hat eine KEM-Zone 45 mit einer inhomogen verteilten Einbettung von Knochenersatzmaterial innerhalb der Kollagenmatrix. Im Unterschied zu den vorherigen Ausführungsbeispielen hat der Formkörper aber keine kreisrunde, sondern eine ovale Querschnittsfläche. Sein Längsschnitt sieht dagegen genauso aus wie bei dem Ausführungsbeispiel gemäß Fig. 9. Grundsätzlich können auch die anderen Ausführungsbeispiele von Formkörpern anstelle der runden eine ovale Querschnittsfläche senkrecht zur Mittenlängsachse 6 haben.

Das in Fig. 11 gezeigte weitere Ausführungsbeispiel eines Formkörpers 26 ähnelt dem des Formkörpers 14 gemäß Fig. 5. Der Formkörper 26 hat einen kegelstumpfförmigen Grundkörper 27, auf dessen oberer Randfläche 28 eine Abdeckmembran 29 angeordnet ist. Der Grundkörper 27 entspricht im Wesentlichen dem Formkörper 14 gemäß Fig. 5, wobei hierin keine Einschränkung zu sehen ist. Im Prinzip könnte jeder andere der in den Fig. 1 bis 10 gezeigten Formkörper 1, 7, 10, 12, 15, 16, 23, 39 und 44 mit einer der Abdeckmembran 29 vergleichbaren Abdeckung versehen sein. Die Abdeckmembran 29 überragt die Randfläche 28 überall. Sie ist fest mit dem Grundkörper 27 verbunden. Die Wirkungsweise und besondere Ausgestaltungen der Abdeckmembran 29 sind in der DE 10 2008 010 893 B3 beschrieben.

Die in den Figuren 1 bis 11 dargestellten Formkörper 1, 7, 10, 12, 14, 15, 16, 23, 26, 39 und 44 sind jeweils unter Zuhilfenahme des kollagenhaltigen Kompositmaterials hergestellt. Sie haben jeweils einen Anteil an Knochenersatzmaterial. Trotzdem sind sie komprimierbar und lassen sich deshalb einfach in eine Knochendefektstelle einsetzen, ohne dabei Folgeverletzungen an den Wänden der Knochendefektstelle hervorzurufen.

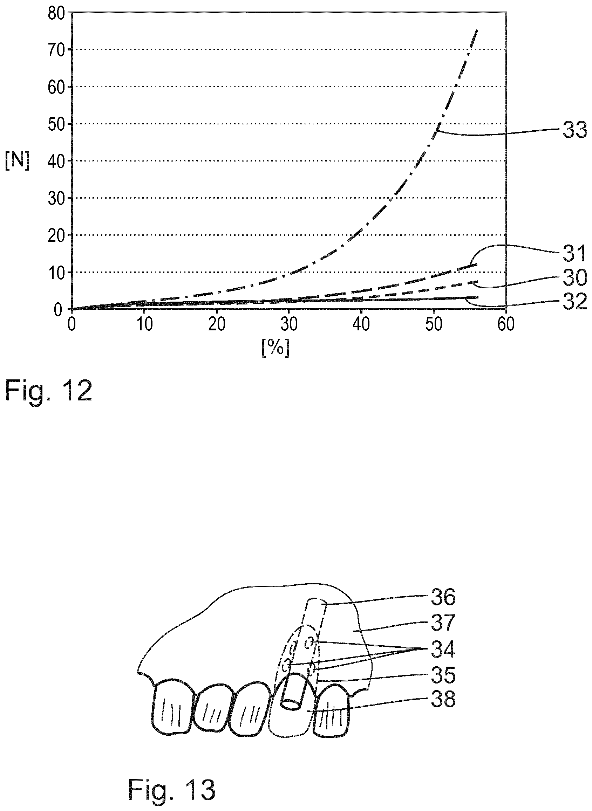

Die gute Komprimierbarkeit wird anhand der in Fig. 12 gezeigten Messkurven dokumentiert. Die Messungen wurden anhand von zwei Formkörpern 14 gemäß Fig. 5 vorgenommen. Bei dem ersten Formkörper 14 enthielt die KEM-Zone 17 insgesamt 0,1 g Knochenersatzmaterial (siehe Messkurve 30 mit kurz gestrichelter Linienführung), wohingegen bei dem zweiten Formkörper 14 die KEM-Zone 17 insgesamt 0,2 g Knochenersatzmaterial enthielt (siehe Messkurve 31 mit lang gestrichelter Linienführung). In dem Diagramm gemäß Fig. 12 ist außerdem eine erste Vergleichsmesskurve 32 (durchgezogene Linienführung) für einen ausschließlich aus Kollagenmaterial bestehenden ersten Vergleichsformkörper eingetragen. Eine zweite Vergleichsmesskurve 33 (strichpunktierte Linienführung) wurde für einen zweiten Vergleichsformkörper ermittelt, in dem eine Gesamtmenge von 0,3 g Knochenersatzmaterial homogen verteilt eingebettet war. Der zweite Vergleichsmesskörper enthielt also insbesondere keine knochenersatzmaterialfreie Zone. Seine Knochenersatzmaterial-Konzentration war im Wesentlichen überall gleich groß.

In dem Diagramm gemäß Fig. 12 ist der Verlauf der Druckkraft über der auf das Ausgangsvolumen bezogenen Kompression, um die der betreffende Formkörper komprimiert wurde, aufgetragen. Dem Messdiagramm ist zu entnehmen, dass die beiden untersuchten Formkörper 14 (Messkurven 30 und 31) bis zu einer 30%igen Kompression ein fast identisches Kompressionsverhalten aufweisen wie der ausschließlich aus Kollagenmaterial bestehende erste Vergleichsformkörper (Messkurve 32). Demgegenüber ist bei dem zweiten Vergleichsformkörper mit homogen eingebettetem Knochenersatzmaterial (Messkurve 33) bereits bei einer Kompression um 7 % eine spürbar höhere Druckkraft erforderlich. Aufgrund der inhomogen verteilten Einbettung des Knochenersatzmaterials wird das Kompressionsverhalten bei den Formkörpern 14 bis zu einer Kompression um etwa 40 % nicht wesentlich beeinträchtigt. Im Gegensatz dazu hat der zweite Vergleichsformkörper mit homogen eingelagertem Knochenersatzmaterial eine deutlich schlechtere Komprimierbarkeit.

Insofern bietet der Formkörper 14 ebenso wie die übrigen in den Figuren 1 bis 11 dargestellten Formkörper 1, 7, 10, 12, 15, 16, 23, 26, 39 und 44 verglichen mit einem Formkörper mit homogen eingelagertem Knochenersatzmaterial auch deutliche Vorteile bei der Handhabung, insbesondere bei dem Einsetzen in die Knochendefektstelle ebenso wie bei dem Anschmiegen an die Wand der Knochendefektstelle. Letzteres begünstigt auch eine besonders vorteilhafte Wundheilung und Knochenregeneration.

Die Formkörper 1, 7, 10, 12, 14, 15, 16, 23, 26, 39 und 44 können in ihren jeweiligen Außenabmessungen in etwa an die Größe der Knochendefektstelle angepasst sein. In diesen Ausgestaltungen wird jeweils ein einziger der Formkörper 1, 7, 10, 12, 14, 15, 16, 23, 26, 39 und 44 in die Knochendefektstelle eingesetzt.

Es gibt aber auch einen anderen in Fig. 13 dargestellten Anwendungsfall mit Formkörpern 34 aus kollagenhaltigem Kompositmaterial mit inhomogen verteilt eingebettetem Knochenersatzmaterial. Die Formkörper 34 sind (deutlich) kleiner als die Knochendefektstelle 35 (= Alveole). Bei diesem Anwendungsfall wird unmittelbar nach der Zahnextraktion ein stiftförmiges Implantat 36 in den verbliebenen Kieferknochen 37 eingebracht. Auf das Implantat 36 wird nach dem Heilungsprozess eine Implantatkrone 38 aufgesetzt. Zwischen dem Implantat 36 und den Wänden der Knochendefektstelle 35 bleiben Hohlräume, die mit den Formkörpern 34 aufgefüllt werden, um den Wundheilungsprozess und das Knochenwachstum, insbesondere das Einwachsen des Implantats 36 zu begünstigen. Die Formkörper 34 können einen im Wesentlichen ähnlichen oder gleichen Aufbau wie die Formkörper 1, 7, 10, 12, 14, 15, 16, 23, 39 und 44 haben. Alternativ sind aber auch andere Außenkonturen mit z.B. würfel- oder quaderförmiger Gestalt möglich. Vorteilhafterweise haben auch die Formkörper 34 die günstige Komprimierbarkeit, so dass sie sich gut in die u.U. nur schwer zugänglichen Hohlräume einsetzen lassen. Dort dehnen sie sich wieder aus und füllen die Hohlräume im Wesentlichen vollständig aus, so dass auch das für den Heilungs- und Knochenwachstumsprozess günstige Anschmiegen an die Wände der Wundhöhle (= Knochendefektstelle 35) gegeben ist.

## Patentansprüche

1. Formkörper zum Einbringen in einen Alveolarraum oder in eine andere Knochendefektstelle (35) zur Wundheilung sowie zur Knochenregeneration und -konservierung, wobei
a) für den Formkörper (1; 7; 10; 12; 14; 15; 16; 23; 26; 34; 39; 44) ein Kompositmaterial mit mindestens einer ersten Materialkomponente in Form eines Kollagenmaterials und einer zweiten Materialkomponente in Form eines Knochenersatzmaterials vorgesehen ist,
b) das Kollagenmaterial eine Matrix bildet, in die das Knochenersatzmaterial inhomogen verteilt eingebettet ist, so dass ein auf ein beliebiges Teilvolumen von 30 mm³ bezogener lokaler Kollagenanteil überall mindestens 10 % beträgt und ein auf das Gesamtvolumen bezogener globaler Kollagenanteil im Bereich zwischen 30 % und 95 % liegt,
c) der Formkörper (1; 7; 10; 12; 14; 15; 16; 23; 26; 34; 39; 44) komprimierbar ist und bei einer Kompression um bis zu 40 % seines Ausgangsvolumens ein Kompressionsmodul von höchstens 1,0 MPa hat,
d) ein jeweils auf ein Teilvolumen von 30 mm³ bezogener lokaler Knochenersatzmaterialanteil an einer bei der Applikation der Öffnung der Knochendefektstelle (35) zugewandten oberen Randfläche (3) seinen maximalen Wert hat und ausgehend von der oberen Randfläche (3) nach innen abnimmt, wobei sich eine mit Knochenersatzmaterial versehene KEM-Zone (2; 8; 11; 13; 17; 24; 40; 45) ausgehend von der oberen Randfläche (3) ins Innere erstreckt, und
e) eine knochenersatzmaterialfreie Zone (4) vorhanden ist, die an eine der oberen Randfläche (3) gegenüberliegende untere Randfläche (5) angrenzt.

2. Formkörper nach Anspruch 1, **dadurch gekennzeichnet, dass** der Formkörper (1; 7; 10; 12; 14; 15; 16; 23; 26; 34; 39; 44) bei einer Kompression um bis zu 40 % seines Ausgangsvolumens ein Kompressionsmodul von höchstens 0,5 MPa, vorzugsweise von höchstens 0,25 MPa, hat.

3. Formkörper nach Anspruch 1 oder 2, **dadurch gekennzeichnet, dass** der maximale Wert des auf ein Teilvolumen von 30 mm³ bezogenen lokalen Knochenersatzmaterialanteils an der oberen Randfläche (3) zwischen 10 % und 90 % liegt.

4. Formkörper nach Anspruch 3, **dadurch gekennzeichnet, dass** der maximale lokale Knochenersatzmaterialanteil an einem auf die gesamte obere Randfläche (3) bezogenen Flächenanteil von zwischen 10 % und 100 % vorliegt.

5. Formkörper nach Anspruch 3 oder 4, **dadurch gekennzeichnet, dass** die Abnahme des Knochenersatzmaterialanteils in der KEM-Zone (2; 8; 11; 13; 17; 24; 40; 45) kontinuierlich oder mit mindestens einer diskreten Abstufung, vorzugsweise mit mehreren diskreten Abstufungen, oder bereichsweise kontinuierlich und bereichsweise abgestuft erfolgt, insbesondere bis zu einem minimalen lokalen Knochenersatzmaterialanteil zwischen 1 % und 40 %.

6. Formkörper nach einem der Ansprüche 3 bis 5, **dadurch gekennzeichnet, dass** eine längliche Grundform mit einer Längsachse (6) und mit einer senkrecht zur Längsachse (6) angeordneten stirnseitigen Begrenzungsfläche (3) vorgesehen ist, und die stirnseitige Begrenzungsfläche die obere Randfläche (3) ist, von der ausgehend der lokale Knochenersatzmaterialanteil abnimmt.

7. Formkörper nach einem der Ansprüche 3 bis 6, **dadurch gekennzeichnet, dass** die KEM-Zone (2; 8; 11; 13; 17; 24; 40; 45) längs ihrer Ausdehnungsrichtung ins Innere bezogen auf eine mit Knochenersatzmaterial versehene KEM-Ausgangsfläche der oberen Randfläche (3) senkrecht zur Ausdehnungsrichtung Querschnittsflächen hat, die größer oder kleiner als die KEM-Ausgangsfläche oder gleich groß wie die KEM-Ausgangsfläche sind.

8. Formkörper nach einem der Ansprüche 3 bis 7, **dadurch gekennzeichnet, dass** die KEM-Zone (2; 8; 11; 13; 17; 24; 40; 45) im Wesentlichen eine tropfen- oder trichterartige Form oder zumindest teilweise die Form eines Teils eines Rotationshyperboloids hat.

9. Formkörper nach einem der vorhergehenden Ansprüche, **dadurch gekennzeichnet, dass** unterscheiden sich das Kollagenmaterial und das Knochenersatzmaterial erkennbar voneinander, wobei insbesondere mindestens ein Material aus der Gruppe von dem Kollagenmaterial und dem Knochenersatzmaterial gefärbt ist.

10. Formkörper nach einem der vorhergehenden Ansprüche, **dadurch gekennzeichnet, dass** das Knochenersatzmaterial aufbereitetes natives Knochenmaterial, insbesondere gereinigtes Spongiosamaterial, oder synthetisches Knochenersatzmaterial, insbesondere Tricalciumphosphat-Granulat, Hydroxylapatit-Granulat, resorbierbares Biokeramik-Granulat, bi- oder multiphasisches Knochenersatzmaterial-Granulat, ist, wobei das Knochenersatzmaterial jeweils insbesondere körnig ist und einen bevorzugten Partikeldurchmesser zwischen 0,05 mm und 3 mm, insbesondere zwischen 0,1 mm und 2 mm, vorzugsweise zwischen 0,2 mm und 1 mm, hat.

11. Formkörper nach einem der vorhergehenden Ansprüche, **dadurch gekennzeichnet, dass** der Formkörper (1; 7; 10; 12; 14; 15; 16; 23; 26; 34; 39; 44) eine im Wesentlichen längliche Grundform mit einer Längsachse (6) hat, wobei die Ausdehnung in Richtung der Längsachse (6) zwischen 0,4 cm und 3 cm und die maximale Ausdehnung senkrecht zur Längsachse zwischen 0,3 cm und 2,9 cm liegt

12. Formkörper nach einem der Ansprüche 1 bis 10, **dadurch gekennzeichnet, dass** der Formkörper eine im Wesentlichen würfelförmige Grundform hat, wobei die Ausdehnung in Richtung der drei Würfelachsen jeweils zwischen 0,3 cm und 3 cm liegt.

## Claims

1. Shaped body for introduction into an alveolar space or into another bone defect location (35) for wound healing and for bone regeneration and preservation, wherein
a) a composite material with at least a first material component in the form of a collagen material and a second material component in the form of a bone replacement material is provided for the shaped body (1; 7; 10; 12; 14; 15; 16; 23; 26; 34; 39; 44),
b) the collagen material forms a matrix, in which the bone replacement material is embedded, distributed non-homogeneously, so that a local collagen proportion based on any desired part volume of 30 mm³ is at least 10 % everywhere and a global collagen proportion based on the total volume is in the range of between 30 % and 95 %,
c) the shaped body (1; 7; 10; 12; 14; 15; 16; 23; 26; 34; 39; 44) is compressible and, at a compression by up to 40 % of its starting volume, has a compressive modulus of at most 1.0 MPa, and
d) a local bone replacement material proportion based on a part volume of 30 mm³ has its maximum value at an upper edge face (3) facing the opening of the bone defect location (35) during application and decreases inwardly proceeding from the upper edge face (3), a BRM zone (2; 8; 11; 13; 17; 24; 40; 45) provided with bone replacement material extending into the interior proceeding from the upper edge face (3), and
e) a bone replacement material-free zone (4) is provided, which adjoins a lower edge face (5) opposing the upper edge face (3).

2. Shaped body according to claim 1, **characterized in that** the shaped body (1; 7; 10; 12; 14; 15; 16; 23; 26; 34; 39; 44) at a compression by up to 40 % of its starting volume, has a compressive modulus of at most 0.5 MPa, preferably at most 0.25 MPa.

3. Shaped body according to claim 1 or 2, **characterized in that** the maximum value of the local bone replacement material proportion based on a part volume of 30 mm³ at the upper edge face (3) is between 10 % and 90 %.

4. Shaped body according to claim 3, **characterized in that** the maximum local bone replacement material proportion is present at an area proportion based on the total upper edge face (3) of between 10 % and 100%.

5. Shaped body according to claim 3 or 4, **characterized in that** the reduction in the bone replacement material proportion in the BRM zone (2; 8; 11; 13; 17; 24; 40; 45) takes place continuously or with at least a discrete graduation, preferably with a plurality of discrete graduations, or continuously in regions and graduated in regions, in particular up to a minimum local bone replacement material proportion of between 1 % and 40 %.

6. Shaped body according to any one of claims 3 to 5, **characterized in that** an elongate basic shape with a longitudinal axis (6) and with an end limiting face (3) arranged perpendicular to the longitudinal axis (6) is provided, and the end limiting face is the upper edge face (3), proceeding from which the local bone replacement material proportion decreases.

7. Shaped body according to any one of claims 3 to 6, **characterized in that** the BRM zone (2; 8; 11; 13; 17; 24; 40; 45), along its extent direction into the interior, in relation to a BRM starting face of the upper edge face (3) provided with bone replacement material, perpendicular to the extent direction, has cross sectional faces, which are greater or smaller than the BRM starting face or the same size as the BRM starting face.

8. Shaped body according to any one of claims 3 to 7, **characterized in that** the BRM zone (2; 8; 11; 13; 17; 24; 40; 45) has substantially a drop-like or funnel-like shape or at least partially the shape of a part of a rotational hyperboloid.

9. Shaped body according to any one of the preceding claims, **characterized in that** the collagen material and the bone replacement material recognizably differ from one another, wherein in particular at least one material from the group of the collagen material and the bone replacement material is dyed.

10. Shaped body according to any one of the preceding claims, **characterized in that** the bone replacement material is prepared native bone material, in particular purified spongy bone material, or synthetic bone replacement material, in particular tricalcium phosphate granulate, hydroxylapatite granulate, resorbable bioceramic granulate, biphasic or multiphasic bone replacement material granulate, wherein the bone replacement material is, in each case, in particular grainy and has a preferred particle diameter of between 0.05 mm and 3 mm, in particular between 0.1 mm and 2 mm, preferably between 0.2 mm and 1 mm.

11. Shaped body according to any one of the preceding claims, **characterized in that** the shaped body (1; 7; 10; 12; 14; 15; 16; 23; 26; 34; 39; 44) has a substantially elongate basic shape with a longitudinal axis (6), wherein the extent in the direction of the longitudinal axis (6) is between 0.4 cm and 3 cm and the maximum extent perpendicular to the longitudinal axis is between 0.3 cm and 2.9 cm.

12. Shaped body according to any one of claims 1 to 10, **characterized in that** the shaped body has a substantially cube-shaped basic shape, wherein the extent in the direction of the three cube axes is between 0.3 cm and 3 cm, in each case.

## Revendications

1. Corps de moulage destiné à être introduit dans un espace alvéolaire ou dans un autre emplacement de défectuosité osseuse (35) pour la guérison des plaies ainsi que pour la régénération et la conservation osseuses, dans lequel
a) pour le corps de moulage (1 ; 7 ; 10 ; 12 ; 14 ; 15 ; 16 ; 23 ; 26 ; 34 ; 39 ; 44) est prévu un matériau composite avec au moins un premier matériau composant sous forme d'une matière collagène et un second composant matériau sous forme d'une charge osseuse ;
b) la matière collagène constitue une matrice dans laquelle la charge osseuse est incorporée, distribuée de façon non homogène, de telle sorte qu'une proportion locale de collagène rapportée à un volume partiel quelconque de 30 mm³ s'élève partout à au moins 10 % et qu'une proportion globale de collagène rapportée au volume global se situe dans une plage allant de 30 % à 95 %,
c) le corps de moulage (1 ; 7 ; 10 ; 12 ; 14 ; 15 ; 16 ; 23 ; 26 ; 34 ; 39 ; 44) est compressible et présente, sous une compression allant jusqu'à 40 % de son volume initial, un module de compression d'au plus 1,0 MPa,
d) une proportion locale de matériau de charge osseuse rapportée respectivement à un volume partiel de 30 mm³ présente sa valeur maximale au moment de l'application au niveau d'une surface périphérique supérieure (3) orientée vers l'orifice de la défectuosité osseuse (35), et partant de la surface périphérique supérieure (3) diminue vers l'intérieur, dans lequel une zone de charge osseuse (2 ; 8 ; 11 ; 13 ; 17 ; 24 ; 40 ; 45) pourvue de charge osseuse (*Knochenersatzmaterial,* de charge osseuse) s'étend partant de la surface périphérique supérieure (3) vers l'intérieur, et
e) une zone dépourvue de charge osseuse (4) est disponible qui jouxte une surface périphérique inférieure (5) opposée à la surface périphérique supérieure (3).

2. Corps de moulage selon la revendication 1, **caractérisé en ce que** le corps de moulage (1 ; 7 ; 10 ; 12 ; 14 ; 15 ; 16 ; 23 ; 26 ; 34 ; 39 ; 44) présente, sous une compression allant jusqu'à 40 % de son volume initial, un module de compression d'au plus 0,5 MPa, de préférence d'au plus 0,25 MPa.

3. Corps de moulage selon la revendication 1 ou 2, **caractérisé en ce que** la valeur maximale de la proportion locale de charge osseuse rapportée à un volume partiel de 30 mm³ se situe au niveau de la surface périphérique supérieure (3) entre 10 % et 90 %.

4. Corps de moulage selon la revendication 3, **caractérisé en ce que** la proportion locale maximale de charge osseuse existe au niveau d'une proportion de surface rapportée à la surface périphérique supérieure (3) totale allant de 10 % à 100 %.

5. Corps de moulage selon la revendication 3 ou 4, **caractérisé en ce que** la diminution de la proportion de charge osseuse dans la zone de charge osseuse (2 ; 8 ; 11 ; 13 ; 17 ; 24 ; 40 ; 45) se produit de façon continue ou au moins avec une gradation discrète, de préférence avec plusieurs gradations discrètes, ou partiellement de façon continue ou partiellement de façon graduée, en particulier jusqu'à une proportion minimale locale de charge osseuse allant de 1 % à 40 %.

6. Corps de moulage selon l'une quelconque des revendications 3 à 5, **caractérisé en ce qu'**est prévue une forme de base longitudinale avec un axe longitudinal (6) et avec une surface de délimitation (3) frontale agencée perpendiculairement à l'axe longitudinal (6), et la surface de délimitation frontale est la surface périphérique supérieure (3) à partir de laquelle la proportion de charge osseuse locale diminue.

7. Corps de moulage selon l'une quelconque des revendications 3 à 6, **caractérisé en ce que** la zone de charge osseuse (2 ; 8 ; 11 ; 13 ; 17 ; 24 ; 40 ; 45) présente, le long de sa direction d'extension vers l'intérieur rapporté à une surface de sortie de charge osseuse dotée de charge osseuse de la surface périphérique supérieure (3), perpendiculairement à la direction d'extension, des surfaces transversales qui sont plus grandes ou plus petites que la surface de sortie de charge osseuse ou de la même taille que la surface de sortie de charge osseuse.

8. Corps de moulage selon l'une quelconque des revendications 3 à 7, **caractérisé en ce que** la zone de charge osseuse (2 ; 8 ; 11 ; 13 ; 17 ; 24 ; 40 ; 45) présente sensiblement une forme de goutte ou d'entonnoir ou au moins partiellement la forme d'une partie d'un hyperboloïde de révolution.

9. Corps de moulage selon l'une quelconque des revendications précédentes, **caractérisé en ce que** la matière collagène et la charge osseuse se différencient nettement l'une de l'autre, dans lequel en particulier au moins un matériau du groupe formé par la matière collagène et la charge osseuse est coloré.

10. Corps de moulage selon l'une quelconque des revendications précédentes, **caractérisé en ce que** la charge osseuse est un matériau osseux natif préparé, en particulier un matériau d'os spongieux nettoyé, ou une charge osseuse synthétique, en particulier des granules de tricalcium phosphate, des granules d'hydroxyapatite, des granules de biocéramique résorbables, des granules de charge osseuse bi ou multiphases, dans lequel la charge osseuse est respectivement en particulier granuleuse et présente un diamètre de particule privilégié allant de 0,05 mm à 3 mm, en particulier allant de 0,1 mm à 2 mm, de préférence allant de 0,2 mm à 1 mm.

11. Corps de moulage selon l'une quelconque des revendications précédentes, **caractérisé en ce que** le corps de moulage (1 ; 7 ; 10 ; 12 ; 14 ; 15 ; 16 ; 23 ; 26 ; 34 ; 39 ; 44) présente une forme de base sensiblement longitudinale avec un axe longitudinal (6), dans lequel l'extension en direction de l'axe longitudinal (6) se situe entre 0,4 cm et 3 cm et l'extension maximale perpendiculaire à l'axe longitudinal se situe entre 0,3 cm et 2,9 cm.

12. Corps de moulage selon l'une quelconque des revendications 1 à 10, **caractérisé en ce que** le corps de moulage présente une forme de base sensiblement cubique, dans lequel l'extension en direction des trois axes de cube se situe respectivement entre 0,3 cm et 3 cm.
